# EUROPEAN PATENT APPLICATION

(11) **EP 4 556 458 A1**
(43) Date of publication of application: **21.05.2025**
(21) Application number: 23839497.7
(22) Date of filing: 30.06.2023
(51) Int. Cl.: C07C 63/36, A61K 31/045, A61K 31/136, A61K 31/192, A61K 31/198, A61K 31/4035, A61K 41/00, A61K 49/10, A61P 25/16, C07B 59/00, C07C 11/22, C07C 33/34, C07C 47/12, C07C 211/58, C07C 229/36, C07C 255/04, C07D 209/44

(54) **AROMATIC COMPOUND, MIXTURE, MOLECULAR PROBE FOR HYPERPOLARIZATION, METABOLITE, DIAGNOSTIC AGENT, DERIVATIZATION AGENT, NAPHTHALENE DERIVATIVE, CATECHOL DERIVATIVE, AND COMPOUND**

(30) Priority: 11.07.2022 JP 2022111350
(71) Applicant: OSAKA UNIVERSITY, Suita-shi Osaka 565-0871 (JP); Nagoya Denki Educational Foundation, Aichi 464-8540 (JP); Taiyo Nippon Sanso Corporation, Tokyo 142-8558 (JP)
(72) Inventor: NEGORO, Makoto, Suita-shi, Osaka 565-0871 (JP); MIYANISHI, Koichiro, Suita-shi, Osaka 565-0871 (JP); KAGAWA, Akinori, Suita-shi, Osaka 565-0871 (JP); MORITA, Yasushi, Toyota-shi, Aichi 470-0392 (JP); MURATA, Tsuyoshi, Toyota-shi, Aichi 470-0392 (JP); TERAUCHI, Tsutomu, Tokyo 142-8558 (JP)
(74) Representative: HGF
(86) International application number: PCT/JP2023/024505
(87) International publication number: WO 2024/014325

(57) **Abstract**

An aromatic compound is composed of a stable isotope, in which two adjacent carbon atoms are ¹³C, atomic nuclei of other atoms to which the two adjacent carbon atoms are bonded have a spin quantum number of 0, and a hydrogen atom which has a spin coupling constant and is bonded to the ¹³C nucleus through a carbon atom is substituted with a deuterium atom. In the aromatic compound, a relaxation time of a ¹³C nucleus excited by a DNP device is longer than that in the related art.

## Description

### TECHNICAL FIELD

The present invention relates to an aromatic compound, a mixture, a molecular probe for hyperpolarization, a metabolite, a diagnostic agent, a derivatization agent, a naphthalene derivative, a catechol derivative, and a compound.

Priority is claimed on Japanese Patent Application No. 2022-111350, filed on July 11, 2022, the content of which is incorporated herein by reference.

### BACKGROUND ART

In NMR (nuclear magnetic resonance) spectroscopy and MRI (magnetic resonance imaging), atomic nucleus spins (hereinafter, also simply referred to as nucleus spins) in a substance are precisely controlled under a strong static magnetic field, and valuable information at the molecular level is read out from an electromagnetic wave signal (NMR signal) which is modulated by an interaction between the nucleus spins and the like. The sensitivity of the NMR signal is proportional to the polarization rate, but the Zeeman energy of the nucleus spins is very low even under a strong magnetic field of several teslas (T) to several tens of teslas applied by a superconducting magnet. A dynamic nuclear polarization (DNP) method is used as a method for improving the measurement sensitivity by improving the polarization rate by several digits. However, the polarization will be lost even the nucleus spins which have been highly polarized by the DNP in a few seconds to a few tens of seconds due to the modulation of the magnetic field by molecular motion or interaction between the nuclear spins inside and outside the molecule.

Therefore, in order to perform the NMR spectroscopy and MRI, which take a long time, using the DNP, it is essential to use a nucleus spin having a long relaxation time. As one of the methods for suppressing the mechanism of relaxation of the nucleus spin, there is a method of using a nuclear spin state (hereinafter, also referred to as a long-lived state) that is compatible with an interaction as relaxation mechanisms. As a relaxation mechanism which strongly causes nuclear spin relaxation, there is a nearest-neighbor nucleus spin-dipole interaction, and it is known that a singlet state of the nearest-neighbor nucleus spin pair is a long-lived state with respect to this interaction.

The singlet state can be generated by using radio wave irradiation. So far, a relaxation time of 26 minutes in a ¹⁵N pair in ¹⁵N₂O (Non-Patent Document 1) and a relaxation time of more than 1 hour in a ¹³C pair in a ¹³C₂-naphthalene derivative (Non-Patent Document 2) have been reported. The method is not only used for holding high polarization for a long time obtained by the DNP, but also used as a probe for observing long-term dynamics or a method for detecting weak chemical bonds.

### Citation List

### Non-Patent Documents

Non-Patent Document 1: Giuseppe Pileio, The Long-Lived Nuclear Singlet State of 15N-Nitrous Oxide in Solution, J. Am. Chem. Soc., 2008, 130, 38, 12582-12583
Non-Patent Document 2: Gabriele Stevanato, et al., A Nuclear Singlet Lifetime of More than One Hour in Room-Temperature Solution, Angew. Chem. Int. Ed. 2015, 54, 3740-3743.

### SUMMARY OF INVENTION

### Technical Problem

A MRI apparatus has been widely used in hospitals as an indispensable imaging diagnostic technology. However, the sensitivity is extremely low compared with other analysis methods, and is limited to imaging of ¹H nuclei of water molecules present in a large amount in a living body. As a method for solving the problem, the DNP method has attracted attention. By using the technology, development of a cancer treatment effect determination technology by observing the metabolism of an administered drug has been promoted. In the method, a material obtained by hyperpolarizing a substance with a DNP device is dissolved and administered into the living body, and a ¹³C nucleus of the drug or the like is observed. However, since the relaxation time of the ¹³C nucleus excited by the DNP technology is at most several tens of seconds, the DNP technology is not suitable for long-term observation, and an application range thereof is limited.

The present invention has been made in view of the above-described circumstances, and an object thereof is to provide an aromatic compound in which a relaxation time of a ¹³C nucleus excited by a DNP device is longer than that in the related art.

### Solution to Problem

[1] An aromatic compound composed of a stable isotope,
   in which two adjacent carbon atoms are ¹³C,
   atomic nuclei of other atoms to which the two adjacent carbon atoms are bonded have a spin quantum number of 0, and
   a hydrogen atom which has a spin coupling constant and is bonded to the ¹³C nucleus through a carbon atom is substituted with a deuterium atom.
[2] A mixture containing an aromatic compound composed of a stable isotope, the mixture containing:
   a first aromatic compound in which two adjacent carbon atoms are ¹³C, atomic nuclei of other atoms to which the two adjacent carbon atoms are bonded have a spin quantum number of 0, and a hydrogen atom which has a spin coupling constant and is bonded to the ¹³C nucleus through a carbon atom is substituted with a deuterium atom; and
   a second aromatic compound in which one of two adjacent carbon atoms is ¹³C and the other of the two adjacent carbon atoms is ¹²C, atomic nuclei of other atoms to which the two adjacent carbon atoms are bonded have a spin quantum number of 0, and a hydrogen atom which has a spin coupling constant and is bonded to the ¹³C nucleus through a carbon atom is substituted with a deuterium atom.
[3] A molecular probe for hyperpolarization, containing the aromatic compound according to [1].
[4] A metabolite containing the aromatic compound according to [1].
[5] A diagnostic agent containing the aromatic compound according to [1].
[6] A derivatization agent containing the aromatic compound according to [1].
[7] A compound generated by bonding the derivatization agent according to [6] to a diagnostic agent or a metabolite.
[8] A naphthalene derivative represented by Formula (1), 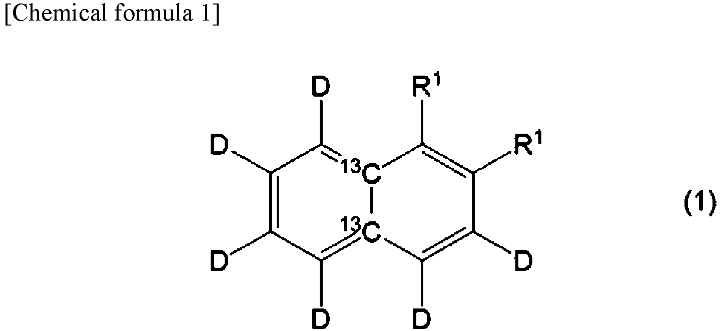 in Formula (1), R¹'s are each independently any one group selected from the group consisting of a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide.
[9] A compound generated by bonding the naphthalene derivative according to [8] to a diagnostic agent or a metabolite.
[10] A catechol derivative represented by Formula (2), 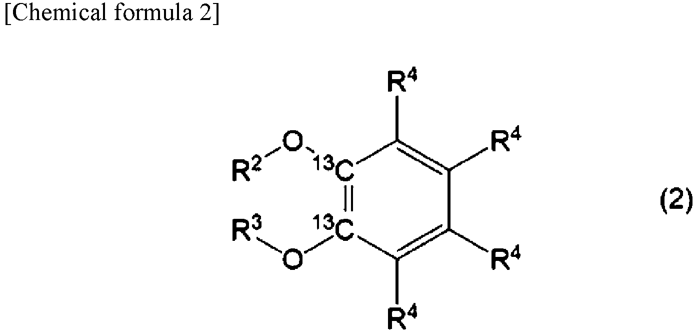 in Formula (2), R² and R³ are each independently any one group selected from the group consisting of a hydrogen atom, a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide, and R⁴'s are each independently any one group selected from the group consisting of a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide, provided that at least one of R⁴'s is a deuterium atom.
[11] A compound generated by bonding the catechol derivative according to [10] to a diagnostic agent or a metabolite.

### Advantageous Effects of Invention

The present invention provides an aromatic compound in which a relaxation time of a ¹³C nucleus excited by a DNP device is longer than that in the related art.

### BRIEF DESCRIPTION OF DRAWINGS

[FIG. 1] FIG. 1 is a diagram representing a result of a ¹³C NMR spectrum measurement of 2-naphthoic acid-4a,8a-¹³C₂ (¹³C₂-NA-h₇).
[FIG. 2] FIG. 2 is a diagram representing a pulse sequence used for generating, holding, and observing a long-lived state.
[FIG. 3] FIG. 3 is a graph representing measurement results of longitudinal relaxation times of 2-naphthoic acid-4a,8a-¹³C₂ (¹³C₂-NA-h₇) and ¹³C₂-2-naphthoic acid-d₇ (¹³C₂-NA-d₇).
[FIG. 4] FIG. 4 is a graph representing measurement results of relaxation times in a singlet state of 2-naphthoic acid-4a,8a-¹³C₂ (¹³C₂-NA-h₇) and 2-naphthoic acid-4a,8a-¹³C₂-d₇ (¹³C₂-NA-d₇).

### DESCRIPTION OF EMBODIMENTS

Hereinafter, embodiments of the present invention will be described in more detail, but the present invention is not limited to the embodiments described below and various modifications can be made without departing from the gist of the present invention.

Meanings and definitions of terms in the present specification are as follows.

A numerical range represented by "to" means a numerical range in which numerical values before and after "to" are the lower limit value and the upper limit value.

"D" used in a chemical formula represents a deuterium atom.

By using a long-lived state of two ¹³C nucleus spin pairs in cyclo condensed carbons of a naphthoic acid skeleton, it was possible to obtain a polarization lifetime of approximately 5 times as long as a longitudinal relaxation time, and approximately 13 times as long as a longitudinal relaxation time by deuterating a hydrogen atom of the aromatic ring. By using a molecular dynamical method and quantum chemical calculation, a simulation of the relaxation time is also performed, and the relaxation time of the molecule in a case where other substituents are added to the naphthoic acid skeleton is predicted, and thus it is confirmed that the relaxation time can be predicted in the same order as the experimental results. From this result, a long-lived nuclear polarization probe having functionality by imparting various substituents to the naphthoic acid skeleton is proposed.

### [Aromatic compound]

The aromatic compound according to the embodiment of the present invention is an aromatic compound composed of a stable isotope, in which two adjacent carbon atoms are ¹³C, atomic nuclei of other atoms to which the two adjacent carbon atoms are bonded have a spin quantum number of 0, and a hydrogen atom which has a spin coupling constant and is bonded to the ¹³C nucleus through a carbon atom is substituted with a deuterium atom.

Specifically, the aromatic compound satisfying the above-described conditions needs to have a ¹³C pair constituting a part of an aromatic ring, and an atom other than a hydrogen atom is bonded to the ¹³C nuclei. For example, a compound in which a bridgehead site at a cyclo condensed position of a polycyclic aromatic compound such as naphthalene, anthracene, phenanthrene, anthraquinone, and a derivative thereof is substituted with ¹³C, or an aromatic compound in which a carbon atom to which a substituent such as o-xylene, o-cresol, catechol, polyphenol, phthalic acid, and a derivative thereof is bonded is substituted with 13C corresponds to the above.

**In** addition, in order to achieve a long polarization lifetime, it is desirable that hydrogen atoms in the vicinity of the ¹³C nuclei be substituted with an atomic nucleus having a spin quantum number of 0 for the purpose of suppressing relaxation due to dipole interaction or the like. The present inventors have found, through theoretical calculations and experiments, that deuterating hydrogen atoms, which are in the vicinity of the ¹³C nuclei to the extent of spin-coupling, can achieve a sufficiently long relaxation time, and have completed the present invention.

As specific examples of the aromatic compound according to the embodiment of the present invention, a naphthalene derivative represented by Formula (1) and a catechol derivative represented by Formula (2) are exemplary examples.

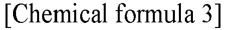

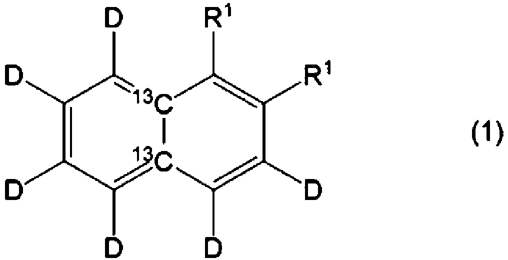

**In** Formula (1), R¹'s are each independently any one group selected from the group consisting of a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide.

As the above-described alkyl group, for example, an alkyl group having 1 to 10 carbon atoms is an exemplary example. It is preferable that the molecular weight of the compound represented by Formula (1) be smaller; and as the above-described alkyl group, an alkyl group having 2 or less carbon atoms, that is, a methylene group or an ethylene group is preferable, and a methylene group is more preferable.

As the above-described aryl group, for example, an aryl group having 6 to 13 carbon atoms is an exemplary example. It is preferable that the molecular weight of the compound represented by Formula (1) be smaller; and as the above-described aryl group, an aryl group having 10 or less carbon atoms is preferable, and a naphthyl group or a phenyl group is more preferable.

The above-described alkene is not particularly limited as long as it is a compound having an alkene in the molecule.

The above-described alkyne is not particularly limited as long as it is a compound having an alkyne in the molecule.

The above-described amine is not particularly limited as long as it is a compound having an amine in the molecule.

The above-described silyl is not particularly limited as long as it is a compound having a silyl in the molecule.

The above-described carbonyl compound is not particularly limited as long as it is a compound having a carbonyl group in the molecule.

The above-described ether is not particularly limited as long as it is a compound having an ether bond in the molecule.

The above-described thiol is not particularly limited as long as it is a compound having a thiol group in the molecule.

The above-described ester is not particularly limited as long as it is a compound having an ester bond in the molecule. Specifically, for example, compounds obtained by dehydration condensation of carbonic acid, nitric acid, phosphoric acid, sulfuric acid, boric acid, or sulfonic acid with an alcohol are exemplary examples.

The above-described sulfoxide is not particularly limited as long as it is a compound in which two carbon atoms in the molecule are bonded to a sulfinyl group (-S(=O)-).

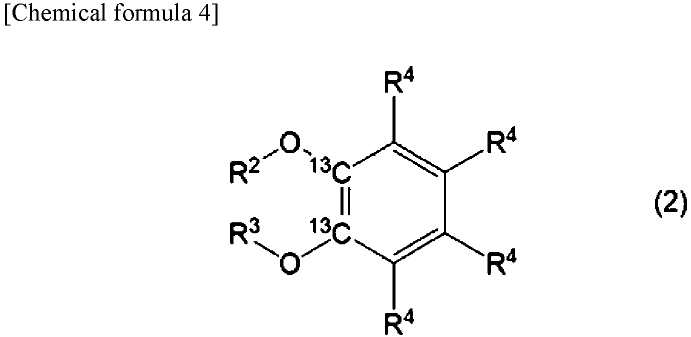

In Formula (2), R² and R³ are each independently any one group selected from the group consisting of a hydrogen atom, a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide.

As the above-described alkyl group, for example, an alkyl group having 1 to 10 carbon atoms is an exemplary example. As the molecular weight of the compound represented by Formula (2) becomes smaller, the relaxation time becomes longer, so that, as the above-described alkyl group, an alkyl group having 2 or less carbon atoms, that is, a methyl group or an ethyl group is preferable, and a methyl group is more preferable.

As the above-described aryl group, for example, an aryl group having 6 to 13 carbon atoms is an exemplary example. As the smaller the molecular weight of the compound represented by Formula (2), the longer the relaxation time, as the above-described aryl group, an aryl group having 10 or less carbon atoms is preferable, and a naphthyl group or a phenyl group is more preferable.

The above-described alkene is not particularly limited as long as it is a compound having an alkene in the molecule.

The above-described alkyne is not particularly limited as long as it is a compound having an alkyne in the molecule.

The above-described amine is not particularly limited as long as it is a compound having an amine in the molecule.

The above-described silyl is not particularly limited as long as it is a compound having a silyl in the molecule.

The above-described carbonyl compound is not particularly limited as long as it is a compound having a carbonyl group in the molecule.

The above-described ether is not particularly limited as long as it is a compound having an ether bond in the molecule.

The above-described thiol is not particularly limited as long as it is a compound having a thiol group in the molecule.

The above-described ester is not particularly limited as long as it is a compound having an ester bond in the molecule. Specifically, for example, compounds obtained by dehydration condensation of carbonic acid, nitric acid, phosphoric acid, sulfuric acid, boric acid, or sulfonic acid with an alcohol are exemplary examples.

The above-described sulfoxide is not particularly limited as long as it is a compound in which two carbon atoms in the molecule are bonded to a sulfinyl group (-S(=O)-).

**In** Formula (2), R⁴'s are each independently any one group selected from the group consisting of a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide. However, at least one of R⁴'s is a deuterium atom.

As the above-described alkyl group, for example, an alkyl group having 1 to 10 carbon atoms is an exemplary example. As the molecular weight of the compound represented by Formula (2) becomes smaller, the relaxation time becomes longer, so that, as the above-described alkyl group, an alkyl group having 2 or less carbon atoms, that is, a methyl group or an ethyl group is preferable, and a methyl group is more preferable.

As the above-described aryl group, for example, an aryl group having 6 to 13 carbon atoms is an exemplary example. As the smaller the molecular weight of the compound represented by Formula (2), the longer the relaxation time, as the above-described aryl group, an aryl group having 10 or less carbon atoms is preferable, and a naphthyl group or a phenyl group is more preferable.

The above-described alkene is not particularly limited as long as it is a compound having an alkene in the molecule.

The above-described alkyne is not particularly limited as long as it is a compound having an alkyne in the molecule.

The above-described amine is not particularly limited as long as it is a compound having an amine in the molecule.

The above-described silyl is not particularly limited as long as it is a compound having a silyl in the molecule.

The above-described carbonyl compound is not particularly limited as long as it is a compound having a carbonyl group in the molecule.

The above-described ether is not particularly limited as long as it is a compound having an ether bond in the molecule.

The above-described thiol is not particularly limited as long as it is a compound having a thiol group in the molecule.

The above-described ester is not particularly limited as long as it is a compound having an ester bond in the molecule. Specifically, for example, compounds obtained by dehydration condensation of carbonic acid, nitric acid, phosphoric acid, sulfuric acid, boric acid, or sulfonic acid with an alcohol are exemplary examples.

The above-described sulfoxide is not particularly limited as long as it is a compound in which two carbon atoms in the molecule are bonded to a sulfinyl group (-S(=O)-).

In the compound represented by Formula (2), oxygen atoms are each independently selected from the group consisting of ¹⁶O, ¹⁷O, and ¹⁸O. Natural abundance ratios of ¹⁶O, ¹⁷O, and ¹⁸O are each 99.757 atm%, 0.038 atm%, and 0.205 atm%.

As the compound represented by Formula (2), specifically, compounds represented by the following formulae are exemplary examples.

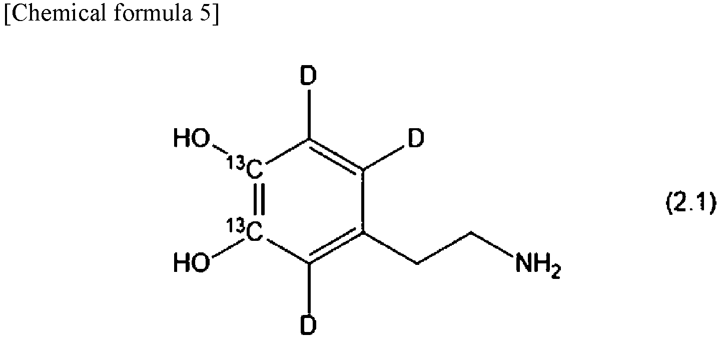

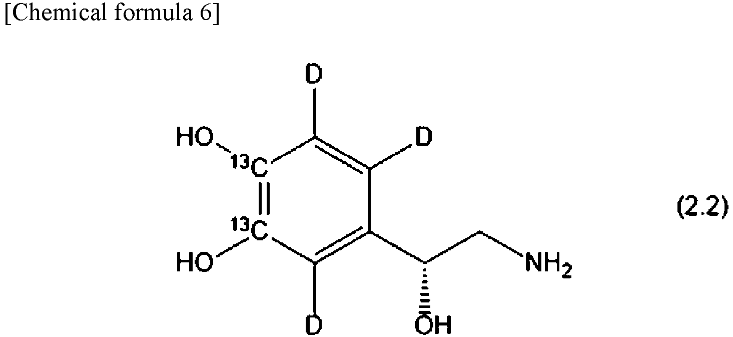

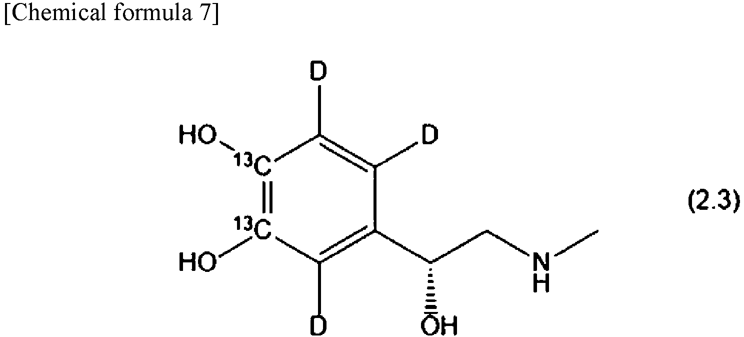

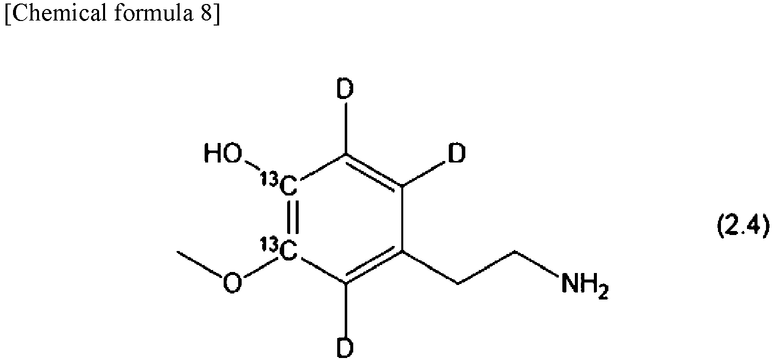

### [Mixture]

The mixture according to the embodiment of the present invention is a mixture containing an aromatic compound composed of a stable isotope, the mixture containing a first aromatic compound in which two adjacent carbon atoms are ¹³C, atomic nuclei of other atoms to which the two adjacent carbon atoms are bonded have a spin quantum number of 0, and one or more hydrogen atoms in a molecule are substituted with deuterium atoms; and a second aromatic compound in which one of two adjacent carbon atoms is ¹³C and the other of the two adjacent carbon atoms is ¹²C, atomic nuclei of other atoms to which the two adjacent carbon atoms are bonded have a spin quantum number of 0, and one or more hydrogen atoms in a molecule are substituted with deuterium atoms.

The above-described first aromatic compound is the compound according to the embodiment of the present invention described above.

The above-described second aromatic compound is synthesized as a by-product in a case of synthesizing the first aromatic compound, because natural abundance ratios of ¹²C and ¹³C are each 98.93 atm% and 1.07 atm%.

### [Molecular probe for hyperpolarization]

The molecular probe for hyperpolarization according to the embodiment of the present invention contains the aromatic compound according to the embodiment of the present invention described above. After hyperpolarization of the molecular probe for hyperpolarization according to the embodiment of the present invention by a DNP device and the administration to a living body or the like, an accumulation state of the molecular probe for long polarization and metabolic conversion of a metabolite thereof can be detected in real time by measurement by a nuclear magnetic resonance method.

### [Metabolite]

The metabolite according to the embodiment of the present invention is a metabolite containing the aromatic compound according to the embodiment of the present invention described above. Hyperpolarization technology enables an overwhelming increase in sensitivity for a specific isotope-labeled probe. However, the nucleus-polarized molecule rapidly relaxes and loses the polarization state due to interaction between the molecular structure and the surrounding environment. Since the present technology can suppress this relaxation phenomenon, by hyperpolarizing the aromatic compound according to the embodiment of the present invention with a DNP device and then dissolving and administering the hyperpolarized aromatic compound in a living body, it is possible to measure metabolic conversion of the aromatic compound or a metabolite thereof in real time.

As specific examples of the metabolite according to the embodiment of the present invention, compounds represented by the following formulae are particularly preferable.

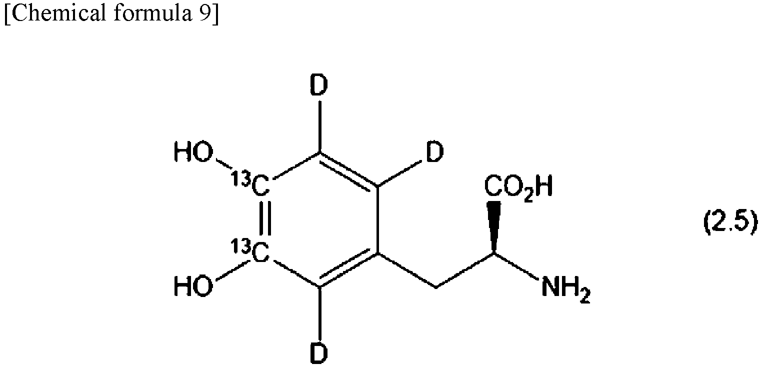

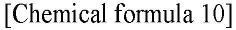

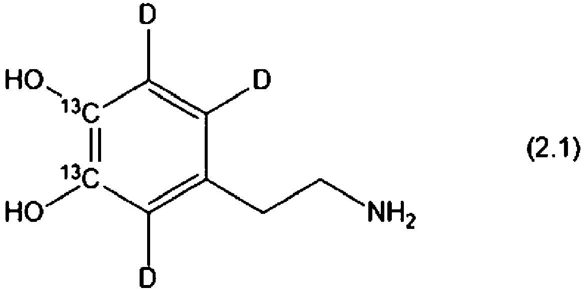

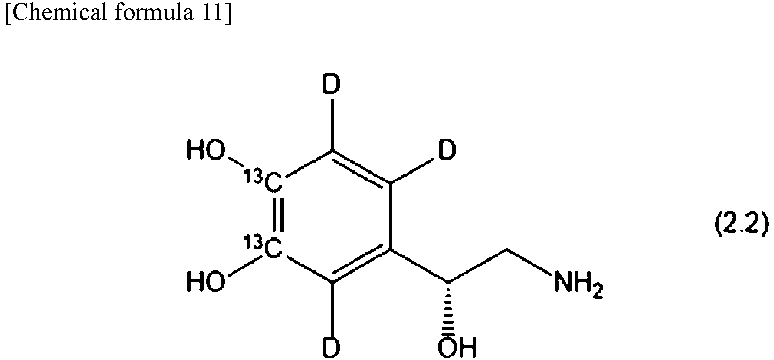

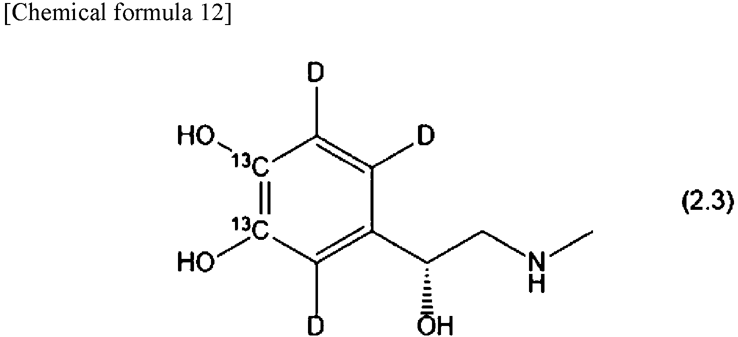

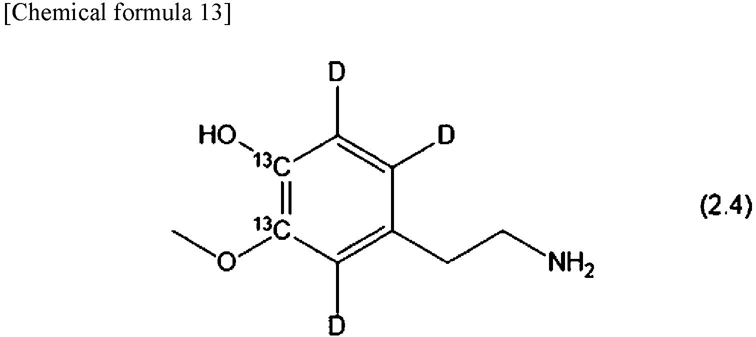

### [Diagnostic agent]

The diagnostic agent according to the embodiment of the present invention is a diagnostic agent containing the aromatic compound according to the embodiment of the present invention described above. The diagnostic agent according to the embodiment of the present invention is hyperpolarized by a DNP device, dissolved, administered to a living body, and then accumulated in a state of maintaining a polarization state at a specific site such as a lesion of cancer in the body of a human, whereby the metabolic process thereof can be tracked with high sensitivity. As a result, it is possible to detect not only differentiation between a normal tissue and a tumor, but also to predict a prognosis, evaluate the reactivity to the drug efficacy, and the like even before the onset at an earlier stage than in the related art. DOPA has already been used for the treatment of Parkinson's disease, and for example, in a case where the accumulation of DOPA in the brain and the metabolic conversion thereof can be imaged in real time with the labeling pattern described in the present invention, it can also be used for research aimed at understanding the pathology. **In** addition, the aromatic compound according to the embodiment of the present invention is bonded to a therapeutic agent or a diagnostic agent, which is difficult to label with a stable isotope for extending the lifetime, and thus it can be used for a medical technology that integrates treatment and diagnosis with a molecule to which the aromatic compound is bonded.

As specific examples of the diagnostic agent according to the embodiment of the present invention, compounds represented by the following formulae are particularly preferable.

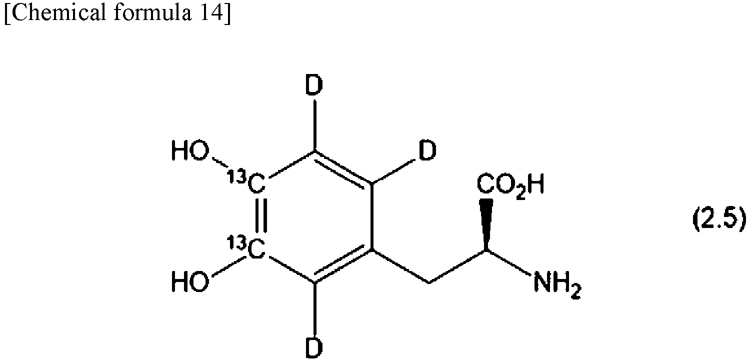

### [Derivatization agent]

The derivatization agent according to the embodiment of the present invention is a derivatization agent containing the aromatic compound according to the embodiment of the present invention described above.

The derivatization agent according to the embodiment of the present invention may further contain at least one compound selected from the group consisting of a carbamate compound, an isothiocyanate compound, an N-hydroxysuccinimide ester, an N-hydroxyphthalimide ester, a pyrylium compound, an acid chloride, an acyl halide, and a sulfonyl halide.

**In** the derivatization agent according to the embodiment of the present invention, the aromatic compound according to the embodiment of the present invention is bonded to a metabolite, a diagnostic agent, or the like, which is difficult to label with a stable isotope for extending the lifetime, and thus it is possible to observe dynamics, interaction, and the like of the molecule to which the aromatic compound is bonded. Examples

Hereinafter, the present invention will be described in more detail with reference to Examples, but the present invention is not limited to Examples described below.

### [Example 1]

A molecule in which two ¹³C (portions indicated by black circle in Formula (3)) in fused ring carbons of carbon atoms of 2-naphthoic acid were substituted with ¹³C, which is an isotope, was synthesized.

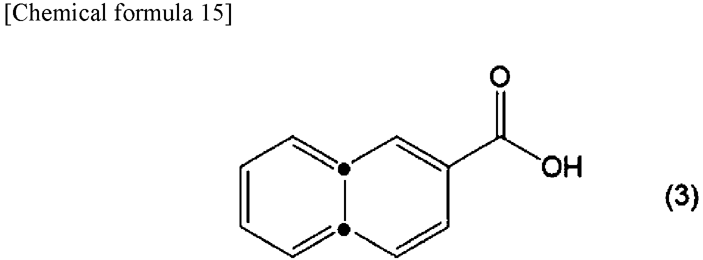

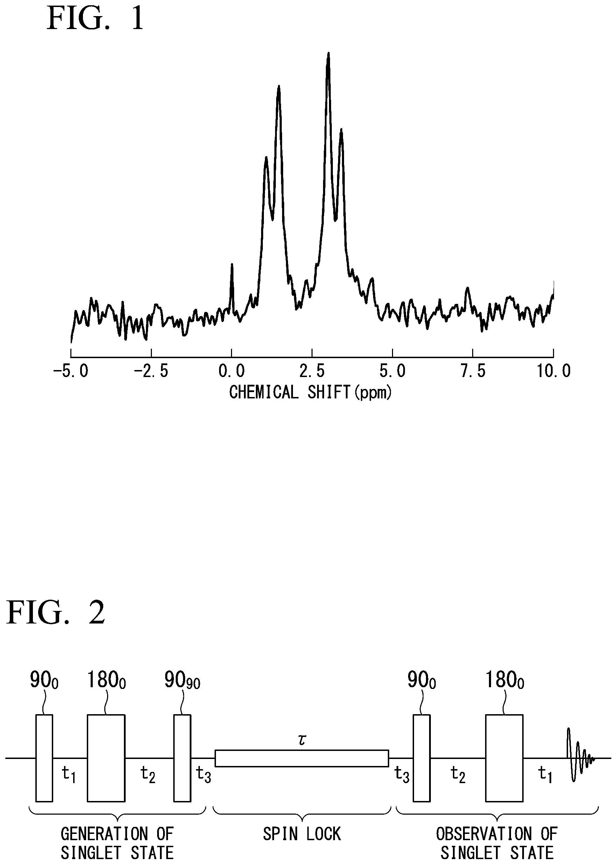

The synthesized 2-naphthoic acid-4a,8a-¹³C₂ (¹³C₂-NA-h₇) was dissolved in deuterium containing 0.3 M potassium carbonate to 0.1 M, and degassed with nitrogen for 30 minutes. FIG. 1 shows a ¹³C NMR spectrum measurement result of the sample, obtained with a 500 MHz NMR device.

From the result shown in FIG. 1, a J-coupling magnitude between the two ¹³C nucleus spins was estimated to be approximately 43 Hz, and a difference in chemical shift was estimated to be approximately 245 Hz.

FIG. 2 shows a pulse sequence used for generating, holding, and observing a long-lived state. From the J-coupling and the difference in chemical shift, it was calculated that t₁ = 4.8 ms, t₂ = 7 ms, and t₃ = 1.1 ms. The spin-locking pulse strength was set to 1.5 kHz, which was sufficiently larger than the difference in chemical shift. As a result of measuring a relaxation time (Ts) in a singlet state using the pulse sequence, it was 34.9 seconds. The longitudinal relaxation time (T₁) obtained by using the inversion recovery method was 7.2 seconds, and it was possible to obtain a relaxation time of approximately 5 times by using the singlet state.

In order to further suppress the intramolecular dipole-dipole interaction, 2-naphthoic acid-4a,8a-¹³C₂-d₇ (¹³C₂-NA-d₇) (Formula (4)) in which the hydrogen atom of the aromatic ring was deuterated was synthesized.

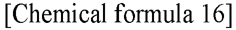

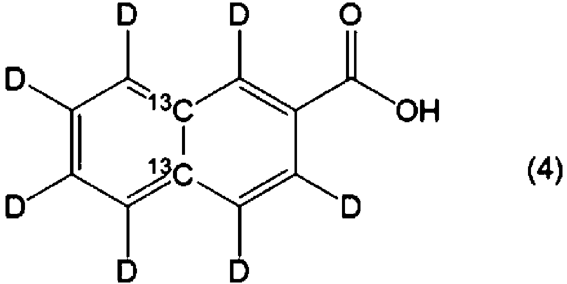

In a case where the longitudinal relaxation time was measured for ¹³C₂-NA-d₇ using the same pulse sequence (FIG. 1), the longitudinal relaxation time was 8.1 seconds, and the relaxation time in the singlet state was 104 seconds, and thus it was possible to obtain a relaxation time of approximately 13 times longer than the longitudinal relaxation time. The measurement was performed under a measurement condition of 11.7 T in an external magnetic field at 27°C.

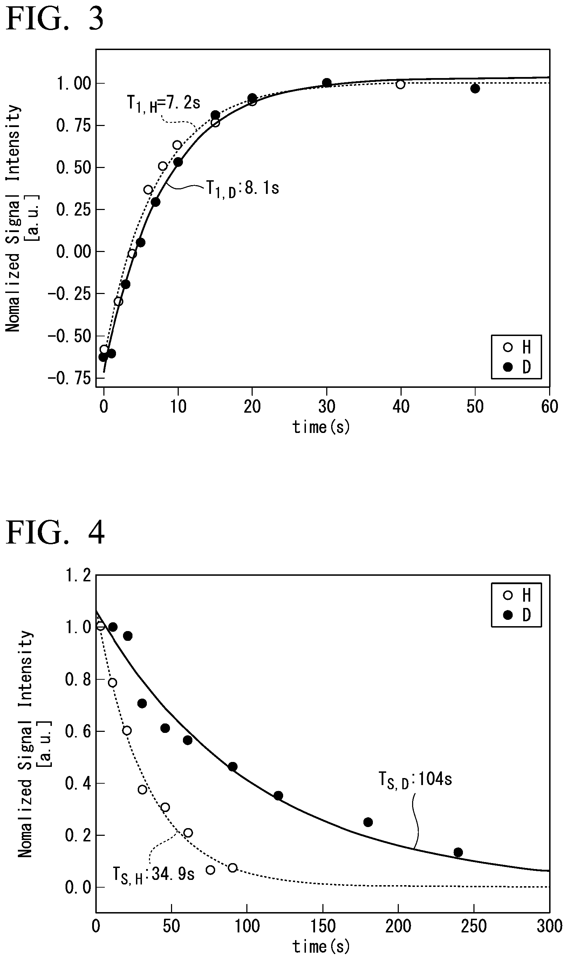

FIG. 3 shows measurement results of the longitudinal relaxation times of ¹³C₂-NA-h₇ (dotted line) and ¹³C₂-NA-d₇ (solid line); and FIG. 4 shows measurement results of the relaxation times in the singlet state of ¹³C₂-NA-h₇ (dotted line) and ¹³C₂-NA-d₇ (solid line).

With the above-described experiments, a relaxation time simulation using quantum chemical calculation and a molecular dynamical method was also performed. The results of the longitudinal relaxation time are shown in Table 1, and the results of the singlet state are shown in Table 2. The calculation was performed under a calculation condition of 11.7 T in an external magnetic field at 27°C.

**[Table 1]**

| Molecular name | R_{1, DDjk} (1/s) | R_{1, inDD} (1/s) | R_{1, CSA} (1/s) | R₁ (1/s) | T_{1, cal} (s) | T_{1, exp} (s) |
|---|---|---|---|---|---|---|
| ¹³C₂-NA-h₇ | 0.018 | 0.023 | 0.332 | 0.373 | 2.68 | 7.2 |
| ¹³C₂-NA-d₇ | 0.014 | 0.002 | 0.32 | 0.336 | 2.98 | 8.09 |

**[Table 2]**

| Molecular name | R_{S, DDjk} (1/s) | R_{S, inDD} (1/s) | R_{S, CSA} (1/s) | R_{S} (1/s) | T_{S, cal} (s) | T_{S, exp} (s) |
|---|---|---|---|---|---|---|
| ¹³C₂-NA-h₇ | - | 0.0219 | - | 0.0219 | 45.6 | 34.9 |
| ¹³C₂-NA-d₇ | - | 0.0018 | - | 0.0018 | 556 | 104 |

With regard to Rᵢ in the tables, a relaxation rate (R_{i, DDjk}) due to a dipole-dipole interaction between two nucleus spins forming the singlet state, a relaxation rate (R_{i, inDD}) due to a dipole-dipole interaction derived from nuclear spins present in other solute molecules, and a relaxation rate (R_{i, CSA}) due to a chemical shift anisotropic interaction are indicated with respect to a longitudinal relaxation time (i = 1) and a singlet state (i = S). In addition to these, the dipole interaction with a nucleus spin contained in the solvent molecule and the relaxation rate due to a spin-rotation interaction were also calculated, but since their contribution was several orders of magnitude smaller, they are not described in the tables. By comparing the numerical calculation result with the experimental result, it is found that the relaxation time prediction could be performed in the same order for both the longitudinal relaxation time and the relaxation time in the singlet state.

It was found that almost 90% of the longitudinal relaxation occurred due to the chemical shift anisotropic interaction. It is considered that the relaxation time calculation accuracy is increased by increasing the accuracy of the chemical shift tensor calculation of performing quantum chemical calculation for each state obtained by the molecular dynamics calculation. In addition, in a case of calculating a difference in relaxation rate of the singlet state depending on the presence or absence of deuteration, (1/T_{S,H}) - (1/T_{S,H}) = 0.019. Since the difference in relaxation rate due to the intramolecular interaction predicted by the calculation was 0.020, it is found that the dipole interaction relaxation rate due to the hydrogen atom in the aromatic ring could be estimated with high accuracy. In order to make the relaxation time in the long-lived state longer, it is considered to use a freeze deaeration method instead of nitrogen deaeration, to increase the strength of the spin-locking pulse, and the like.

### [Example 2]

### <Prediction of Relaxation Time of Other Molecules Having Naphthalene Skeleton>

As candidates for a polarization probe having a naphthalene skeleton, relaxation time prediction was performed for a ¹³C spin pair in an aromatic ring of ¹³C₂-2-naphthylamine-d₇ (¹³C₂-NAmin-d₇) represented by Formula (5) and ¹³C₂-2-naphthalenephthalimide ester-d₁₁ (¹³C₂-NX-d₁₁) represented by Formula (6). The calculation was performed under a calculation condition of 11.7 T in an external magnetic field at 27°C.

Since the ¹³C₂-NX-d₁₁ was expected to be insoluble in water, the relaxation time was calculated in acetone. The calculation results of the longitudinal relaxation time are shown in Table 3; and the calculation results of the relaxation time in the singlet state are shown in Table 4. It was possible to obtain a result in which each of the relaxation times in the singlet state was expected to be sufficiently large compared with T₁.

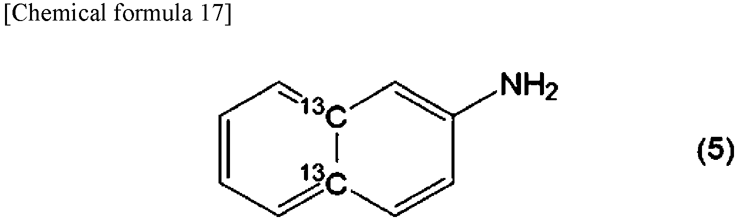

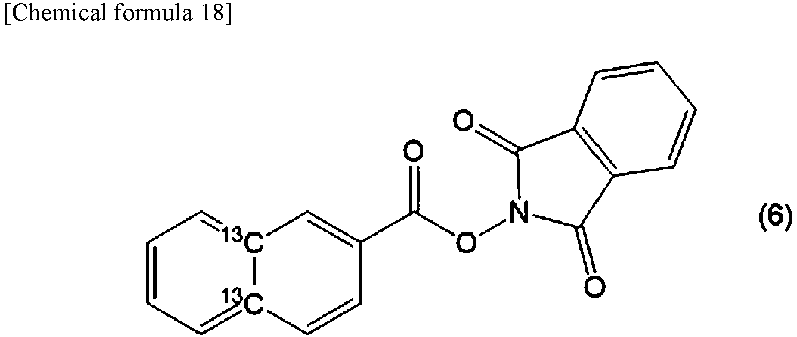

The calculation results of the longitudinal relaxation times of ¹³C₂-2-naphthylamine-d₇ (¹³C₂-NAmin-d₇) and ¹³C₂-2-naphthalenphthalimide ester-d₁₁ (¹³C₂-NX-d₁₁) are shown in Table 3; and the calculation results of the relaxation times of ¹³C₂-2-naphthylamine-d₇ (¹³C₂-NAmin-d₇) and ¹³C₂-2-naphthalenphthalimide ester-d₁₁ (¹³C₂-NX-d₁₁) in the singlet state are shown in Table 4. A component smaller than 10⁻⁴ is indicated by -.

**[Table 3]**

| Molecular name | R_{1, DDjk} (1/s) | R_{1, inDD} (1/s) | R_{1, CSA} (1/s) | R₁ (1/s) | T_{1, cal} (s) |
|---|---|---|---|---|---|
| ¹³C₂-NAmin-d₇ | 0.019 | 0.002 | 0.43 | 0.452 | 2.21 |
| ¹³C₂-NX-d₁₁ | 0.01 | 0.002 | 0.276 | 0.288 | 3.47 |

**[Table 4]**

| Molecular name | R_{S, DDjk} (1/s) | R_{S, inDD} (1/s) | R_{S, CSA} (1/s) | R_{S} (1/s) | T_{S, cal} (s) |
|---|---|---|---|---|---|
| ¹³C₂-NAmin-d₇ | - | 0.0024 | 0.0009 | 0.0034 | 294 |
| ¹³C₂-NX-d₁₁ | - | 0.002 | 0.0001 | 0.0021 | 466 |

### [Example 3]

### <Prediction of Relaxation Time of Other Molecules Having Catechol Skeleton>

As a candidate for a polarization probe having a catechol skeleton, relaxation time prediction was performed for a ¹³C spin pair in an aromatic ring of DOPA-13C2-d6 represented by Formula (7). In addition, aromatic rings and exchangeable hydrogen atoms were replaced with deuterium for the calculations.

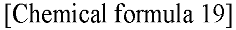

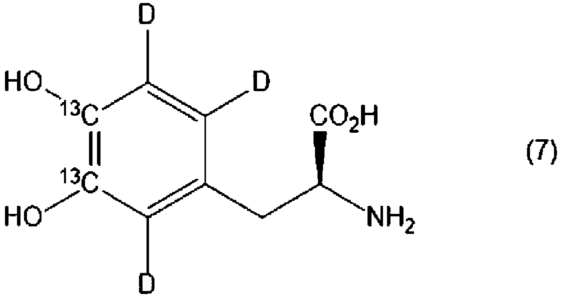

Table 5 shows the calculation result (T₁) of the longitudinal relaxation time and the calculation result (T_{S}) of the relaxation time in the singlet state. In the external magnetic field of 11.7 T, the calculation result of Tₛ was 28.7 seconds; but in a case where the external magnetic field was set to 1 T, the calculation result was 298 seconds, and a result was obtained in which a large relaxation time of the singlet state was expected.

**[Table 5]**

| Magnetic field strength | T₁ (s) | T_{S} (s) |
|---|---|---|
| 11.7 T | 10.8 | 28.7 |
| 1 T | 64.1 | 298 |

### [Example 4]

### <Synthesis of adiponitrile-1,6-¹³C₂>

Potassium cyanide-¹³C (5.820 g, 88.1 mol), ethanol (40 mL), water (20 mL), and 1,4-diiodobutane (6.07 mL, 14.3 g, 46.2 mmol, 0.525 eq) were charged into a 200 mL eggplant flask, and heating under reflux was carried out for 18 hours. After cooling to room temperature, 10 mL of a 2 M sodium hydroxide aqueous solution was added thereto, a pH of the reaction solution was confirmed to be 12 or more, and ethanol was distilled off by concentration under reduced pressure and extraction was carried out with dichloromethane (200 mL × 3). The organic layer was washed with water, and after confirming that cyanide was not present in the water layer, the organic layer was dried with sodium sulfate. The solution was concentrated under reduced pressure and purified by silica gel column chromatography to obtain adiponitrile-1,6-¹³C₂ (yielding amount: 3.986 g (36.2 mmol), yield: 82%).

### <Synthesis of adipaldehyde-1,6-¹³C₂>

Under a nitrogen atmosphere, adiponitrile-1,6-¹³C₂ (5.436 g, 49.4 mol) obtained in the previous reaction and dehydrated dichloromethane (180 mL) were charged into a 500 mL three-neck flask, and the mixture was cooled to -80°C. 1 M DIBAL-H in hexane (105 mL, 105 mol) was added dropwise thereto using a dropping funnel over 50 minutes. After stirring for 2 hours, an additional 1 M DIBAL-H in hexane (35 mL, 35 mol) was added thereto, and the mixture was further stirred for 1 hour. 2 M HCl (150 mL) was slowly added dropwise thereto, the temperature was set to room temperature, and the mixture was stirred for 30 minutes. Subsequently, an aluminum salt was dissolved by dropwise adding 6 M HCl with a Pasteur pipette, and extraction was carried out with dichloromethane (400 mL × 3). The organic layer was washed with saturated saline, dried over sodium sulfate, and concentrated at normal pressure to obtain a crude product 2.

### <Synthesis of 1,1,8,8-tetrabromooctane-2,7-¹³C₂>

Under a nitrogen atmosphere, carbon tetrabromide (53.06 g, 160 mmol), triphenylphosphine (83.93 g, 320 mmol), and dehydrated dichloromethane (700 mL) were charged into a 2 L eggplant flask, and the mixture was stirred at 0°C for 1 hour. Subsequently, a dichloromethane solution (38.4 mmol) of adipaldehyde-1,6-¹³C₂ obtained in the previous reaction was added thereto, and the mixture was stirred for 30 minutes and further stirred at room temperature for 30 minutes. Water (400 mL) was added thereto, and the mixture was extracted with dichloromethane (300 mL × 3). The organic layer was dried over sodium sulfate, and concentrated under reduced pressure to obtain a light yellow solid. The light yellow solid was dissolved in methanol (approximately 300 mL) and extracted with hexane (200 mL × 10). After confirming that the target product was not present in the methanol layer by TLC, the hexane layer was concentrated under reduced pressure to obtain a white solid. The white solid was completely dissolved in dichloromethane (30 to 40 mL), and was precipitated again using a large amount of hexane. The obtained white solid was separated by suction filtration, and the filtrate was concentrated under reduced pressure and purified by silica gel column chromatography to obtain 1,1,8,8-tetrabromooctane-2,7-¹³C₂ (oily, yielding amount: 13.172 g (30.6 mmol), yield: 62%).

### <Synthesis of 1,7-octadiene-2,7-¹³C₂>

Under a nitrogen atmosphere, dehydrated THF (100 mL) and 1,1,8,8-tetrabromooctane-2,7-¹³C₂ (13.172 g, 30.6 mmol) obtained in the previous reaction were charged into a 1 L three-neck flask equipped with a 300 mL dropping funnel and a minus thermometer, and the mixture was cooled to -80°C. LDA (0.8 M, 200 mL, 159 mmol) was charged into the 300 mL dropping funnel, and added dropwise thereto over 90 minutes. After stirring at the same temperature for 1 hour, the temperature was raised to 0°C, and the mixture was further stirred for 1 hour. 1 M HCl (400 mL) was slowly added thereto to terminate the reaction, and the mixture was extracted with diethyl ether (300 mL × 3). The organic layer was washed with a 10 wt% sodium thiosulfate aqueous solution (200 mL × 2), dried over anhydrous sodium sulfate, concentrated by performing a concentration operation at normal pressure, and purified by silica gel column chromatography.

### <Synthesis of 6-(1-hydroxyethyl)-1,2,3,4-tetrahydronaphthalene-4a,8a-¹³C₂>

Under a nitrogen atmosphere, bis(1,5-cyclooctadiene)rhodium(I) tetrafluoroborate (115 mg, 0.282 mmol) 4), dried dichloromethane (10 mL), and (S)-H8-BINAP (178 mg, 0.282 mmol) were charged into a 300 mL eggplant flask, and the mixture was stirred for 10 minutes, the nitrogen atmosphere was replaced with a hydrogen atmosphere, and stirred for 1 hour. After distilling off the dichloromethane under reduced pressure, the reaction solution was replaced with the nitrogen atmosphere again, and dried dichloroethane (90 mL) was added thereto. A mixed reagent was prepared by dissolving 1,7-octadiyne-2,7-¹³C₂ (9.4 mmol) and 3-butyn-2-ol (1.317 g, 18.8 mmol) in dehydrated dichloroethane (4 mL), and added dropwise thereto over 10 minutes and further stirred at room temperature for 18 hours. The reaction solvent was concentrated under reduced pressure and purified by silica gel column chromatography to obtain 6-(1-hydroxyethyl)-1,2,3,4-tetrahydronaphthalene-4a,8a-¹³C₂ (oily, yielding amount: 1.001 g (5.61 mmol), yield: 60%).

### <Synthesis of 2-naphthoic acid-4a,8a-¹³C₂>

Under a nitrogen atmosphere, t-butanol (150 mL), potassium t-butoxide (5.139 g, 45.8 mmol), and iodine (4.362 g, 17.2 mmol) were charged into a 300 mL two-neck flask at room temperature, and the mixture was stirred for 10 minutes. Water (37.9 mL, 17.2 mmol) and 2'-acetonaphthone-4a',8a'-¹³C₂ (5.72 mmol) were added thereto, and the mixture was stirred for 1.5 hours. After the solvent was concentrated under reduced pressure, water (300 mL) was added thereto, and the aqueous layer was washed with dichloromethane (200 mL × 3). A suitable amount of crushed ice was put into the water layer, and 6 M HCl was slowly added until the pH reached 1. The extraction was carried out with dichloromethane (200 mL × 3), and the organic layer was washed with a 10 wt% sodium thiosulfate aqueous solution (200 mL × 2) and saturated saline (200 mL × 1). After drying with anhydrous sodium sulfate, the mixture was concentrated under reduced pressure to obtain a crude product 7 of 2-naphthoic acid-4a,8a-¹³C₂ (light yellow solid, yielding amount: 0.750 g (4.31 mmol), yield: 75%).

### · Reference for pulse sequence

Sarkar R., Vasos P. R. and Bodenhausen G. "Singlet-state exchange NMR spectroscopy for the study of very slow dynamic processes" J. Am. Chem. Soc. 129 328-334 (2007)

· Reference for numerical calculation

Miyanishi K., Mizukami W., Motoyama M., Ichijo N., Kagawa A., Negoro M. and Kitagawa M. "Prediction of 1H singlet relaxation via intermolecular dipolar couplings using the molecular dynamics method" J. Phys. Chem. B 126, 19, 3530-3538 (2022)

### INDUSTRIAL APPLICABILITY

In the present invention, since the relaxation time of the excited molecule is much longer than that in the related art, it is possible to observe a molecular probe such as a drug administered to a human body for a long time. As a result, it is expected that various information such as the degree of progression of a tumor, which is difficult to obtain by the PET diagnostic method, can be obtained. In addition, in basic research and drug discovery research using the DNP, the application of the present method, in which the behavior of molecules or the metabolic process can be observed for a long time, is expected.

## Claims

1. An aromatic compound composed of a stable isotope,
wherein two adjacent carbon atoms are ¹³C,
atomic nuclei of other atoms to which the two adjacent carbon atoms are bonded have a spin quantum number of 0, and
a hydrogen atom which has a spin coupling constant and is bonded to the ¹³C nucleus through a carbon atom is substituted with a deuterium atom.

2. A mixture containing an aromatic compound composed of a stable isotope, the mixture comprising:
a first aromatic compound in which two adjacent carbon atoms are ¹³C, atomic nuclei of other atoms to which the two adjacent carbon atoms are bonded have a spin quantum number of 0, and a hydrogen atom which has a spin coupling constant and is bonded to the ¹³C nucleus through a carbon atom is substituted with a deuterium atom; and
a second aromatic compound in which one of two adjacent carbon atoms is ¹³C and the other of the two adjacent carbon atoms is ¹²C, atomic nuclei of other atoms to which the two adjacent carbon atoms are bonded have a spin quantum number of 0, and a hydrogen atom which has a spin coupling constant and is bonded to the ¹³C nucleus through a carbon atom is substituted with a deuterium atom.

3. A molecular probe for hyperpolarization, comprising:
the aromatic compound according to Claim 1.

4. A metabolite comprising:
the aromatic compound according to Claim 1.

5. A diagnostic agent comprising:
the aromatic compound according to Claim 1.

6. A derivatization agent comprising:
the aromatic compound according to Claim 1.

7. A compound generated by bonding the derivatization agent according to Claim 6 to a diagnostic agent or a metabolite.

8. A naphthalene derivative represented by Formula (1), 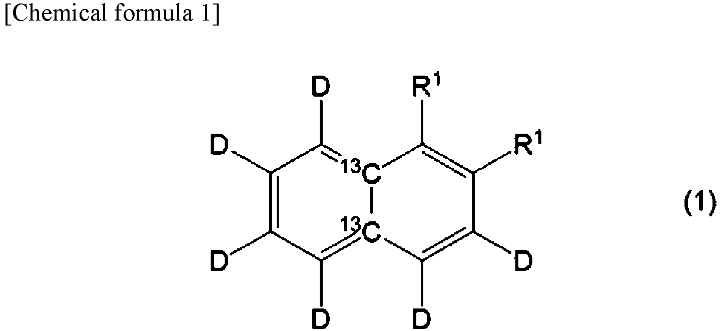 in Formula (1), R¹'s are each independently any one group selected from the group consisting of a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide.

9. A compound generated by bonding the naphthalene derivative according to Claim 8 to a diagnostic agent or a metabolite.

10. A catechol derivative represented by Formula (2), 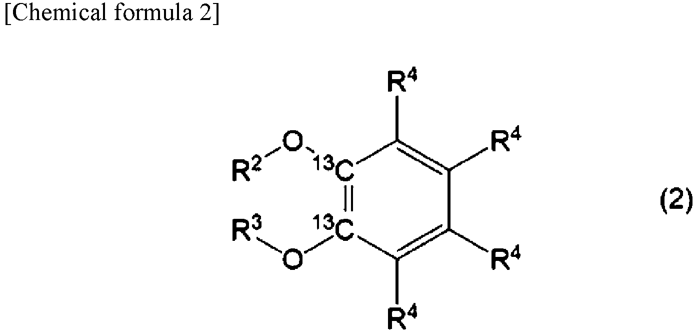 in Formula (2), R² and R³ are each independently any one group selected from the group consisting of a hydrogen atom, a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide, and R⁴'s are each independently any one group selected from the group consisting of a deuterium atom, an alkyl group, an aryl group, and a monovalent group derived from an alkene, an alkyne, an amine, a silyl, a carbonyl compound, an ether, a thiol, an ester, a phosphoric acid ester, or a sulfoxide, provided that at least one of R⁴'s is a deuterium atom.

11. A compound generated by bonding the catechol derivative according to Claim 10 to a diagnostic agent or a metabolite.
